(19) Europäisches Patentamt
European Patent Office
Office européen des brevets

(11) Numéro de publication: **0 490 749 B1**

(12) # FASCICULE DE BREVET EUROPEEN

(45) Date de publication de fascicule du brevet: **01.03.95**

(51) Int. Cl.[6]: **A61K 7/02**

(21) Numéro de dépôt: **91403329.5**

(22) Date de dépôt: **09.12.91**

(54) **Composition cosmétique liquide nettoyante à deux phases, contenant au moins un dialkylphtalate et un agent de surface, anionique, amphotère ou zwittérionique.**

(30) Priorité: **12.12.90 FR 9015579**

(43) Date de publication de la demande:
**17.06.92 Bulletin 92/25**

(45) Mention de la délivrance du brevet:
**01.03.95 Bulletin 95/09**

(84) Etats contractants désignés:
**AT BE CH DE DK ES FR GB GR IT LI NL SE**

(56) Documents cités:
**EP-A- 0 370 856**
**FR-A- 1 526 808**
**FR-A- 2 408 387**
**US-A- 4 767 741**

**CHEMICAL ABSTRACTS, vol. 110, no. 20, 15 mai 1989, page 414, abrégé no.179537v, Columbus, Ohio, US;& JP-A-63 126 543 (SHISEIDO CO., LTD) 30-05-1988**

**CHEMICAL ABSTRACTS, vol. 105, no. 2, 14 juillet 1986, page 109, abrégé no.8633f, Columbus, Ohio, US; &CS-A-225 459 (M. HRBEK et al.) 15-04-1985**

(73) Titulaire: **L'OREAL**
**14, Rue Royale**
**F-75008 Paris (FR)**

(72) Inventeur: **Marion, Hélène**
**75 rue de Patay**
**F-75013 Paris (FR)**
Inventeur: **Louvet, Nathalie**
**13 rue Du Guesclin**
**F-94240 L'Hay-les-Roses (FR)**
Inventeur: **Lukassen, Liliane**
**Résidence de l'Hermitage,**
**24 Allée d'Alsace**
**F-94550 Chevilly-Larue (FR)**

(74) Mandataire: **Casalonga, Axel et al**
**BUREAU D.A. CASALONGA - JOSSE**
**Morassistrasse 8**
**D-80469 München (DE)**

**EP 0 490 749 B1**

Il est rappelé que: Dans un délai de neuf mois à compter de la date de publication de la mention de la délivrance du brevet européen, toute personne peut faire opposition au brevet européen délivré, auprès de l'Office européen des brevets. L'opposition doit être formée par écrit et motivée. Elle n'est réputée formée qu'après paiement de la taxe d'opposition (art. 99(1) Convention sur le brevet européen).

## Description

La présente invention a pour objet une composition liquide nettoyante à deux phases, contenant un ou plusieurs dialkylphtalates et ayant un aspect esthétiquement attrayant.

Le Journal of the American College of Toxicology 1985, Vol. 4, pages 267-271, enseigne l'utilisation d'un dialkylphtalate comme dénaturant de l'alcool et pour fixer les parfums dans les eaux de toilette, les lotions parfumées alcoolisées.

Le document FR-1.526.808 décrit des shampooings comportant des surfactants non-ioniques et des huiles et graisses, le dialkylphtalate étant cité parmi ces huiles et graisses. Le dialkylphtalate, utilisé en présence de surfactants non-ioniques et d'alcools, permet d'obtenir un produit sous forme de crème.

Cependant, la présence de l'alcool est souvent irritante et un besoin existe pour une composition cosmétique aqueuse exempte d'alcool.

On a découvert que la présence d'un dialkylphtalate dans une composition cosmétique liquide aqueuse communique à la composition des qualités cosmétiques appréciables, notamment des propriétés filmogènes et de douceur. En effet, le dialkylphtalate communique à la peau un toucher doux.

On a également constaté que la présence du dialkylphtalate favorise la permanence du parfum dans la composition et sur la peau, même en l'absence d'alcool.

Enfin, l'utilisation du dialkylphtalate permet d'obtenir une composition liquide à deux phases présentant un aspect esthétiquement attrayant.

L'invention a pour objet une composition liquide nettoyante, exempte d'alcool, et comprenant deux phases, une phase aqueuse et une phase huileuse. La phase aqueuse comprend un agent de surface. La phase huileuse comprend de 50 à 100% en poids d'un ou plusieurs dialkylphtalates. Lorsque la teneur en dialkylphtalate de la phase huileuse est inférieure à 100% en poids, le complément à 100% est constitué par un ou plusieurs produits miscibles au dialkylphtalate.

Comme produits miscibles aux dialkylphtalates, on peut citer les huiles, notamment les adipates tels que le dioctyladipate, les myristates tels que l'isopropylmyristate, les palmitates tels que l'octylpalmitate, les stéarates tels que l'isopropylstéarate, des vitamines telles que la vitamine A, la vitamine E, la vitamine F, des huiles telles que l'huile de tournesol, l'huile de poisson, le tétra éthyl-2 hexanoate de pentaérythritol et produits similaires.

On a découvert que lorsque l'agent de surface est choisi parmi les agents de surface anioniques, amphotères, zwittérioniques et leurs mélanges, on obtient des compositions cosmétiques esthétiquement attrayantes se présentant sous forme diphasée.

La phase huileuse contenant le dialkylphtalate se trouve dispersée à l'agitation sous forme de microbilles dans la phase aqueuse, formant une suspension qui, au repos, se dépose au fond du récipient, produisant un effet poudreux esthétiquement plaisant.

Les dialkylphtalates utilisés dans les compositions ont pour formule générale :

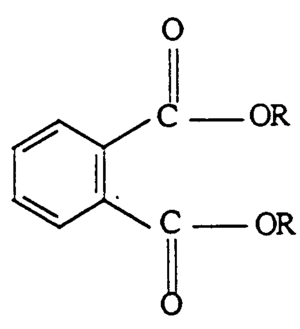

où R est un reste alkyle en $C_1$-$C_4$, en particulier un reste méthyle, éthyle, butyle.

La quantité de dialkylphtalate est de 0,5 à 20% et de préférence de 2 à 15% en poids du poids total de la composition.

La quantité d'agent de surface est de 0,1 à 30% en poids et de préférence de 2 à 6% en poids du poids total de la composition.

La phase aqueuse peut être constituée par de l'eau déminéralisée stérile ou par une eau florale telle que de l'eau de rose, de l'eau de bleuet, de l'eau de camomille ou de l'eau de tilleul.

La phase aqueuse représente de 74 à 99,5% et de préférence de 90 à 95% en poids du poids total de la composition.

Parmi les agents de surface anioniques, on peut citer plus particulièrement les acyliséthionates, les acyl méthyl taurates, les sels alcalins, les sels de magnésium, d'ammonium, d'amines ou d'amino alcools des composés suivants :

- les alkylsulfates, les alkyléthersulfates, les alkylamidoéthersulfates, les alkylarylpolyéthersulfates, les monoglycérides sulfates;
- les alkylsulfonates, les alkylamides sulfonates, les alkylaryl sulfonates, les oléfines sulfonates, les paraffines sulfonates;
- les alkylsulfosuccinates, les alkyléthersulfosuccinates, les alkylamide-sulfosuccinates;
- les alkylsulfosuccinamates, les alkylsulfoacétates;
- les alkylphosphates, les alkylétherphosphates;
- les acylsarcosinates;
- les N-acylpolypeptides.

Le radical alkyle ou acyle de ces différents composés est généralement constitué par une chaîne carbonée comportant de 12 à 20 atomes de carbone.

Parmi les agents de surface anioniques, on peut également citer :

les sels d'acides gras tels que les sels des acides oléique, ricinoléique, palmitique, stéarique; des acides d'huile de coprah ou d'huile de coprah hydrogénée; les acyl lactylates, dont le radical acyle comporte de 8 à 20 atomes de carbone.

Parmi les agents de surface considérés comme faiblement anioniques, on peut citer :

les acides éthers carboxyliques polyoxyalkylénés répondant à la formule :

$$R\text{-}(OC_3H_6)_p\text{-}(OC_2H_4)_n\text{-}OCH_2COOA$$

dans laquelle R désigne un radical alkyle ou un mélange de radicaux alkyle ou alcényle en $C_8$-$C_{22}$, linéaire ou ramifié, un radical alkyl ($C_8$-$C_9$)phényle, R'CONH-$CH_2$-$CH_2$- avec R' désignant un radical alkyle ou alcényle, linéaire ou ramifié, en $C_{11}$-$C_{21}$;

n est un nombre entier ou décimal compris entre 2 et 24,

p est un nombre entier ou décimal compris entre 0 et 6,

A désigne un atome d'hydrogène ou bien Na, K, Li, 1/2 Mg ou un reste monoéthanolamine, ammonium ou triéthanolamine.

Les agents de surface amphotères, zwittérioniques plus particulièrement préférés, sont :

- les dérivés d'amines secondaires ou tertiaires aliphatiques, dans lesquels le radical aliphatique est une chaîne linéaire ou ramifiée comportant de 8 à 18 atomes de carbone et qui contient au moins un groupe anionique hydrosolubilisant carboxylique, sulfonate, sulfate, phosphate ou phosphonate;
- les alkyl($C_8$-$C_{20}$)imidazolinium carboxyméthyle;
- les N-alkyl($C_8$-$C_{20}$)- -iminodipropionates;
- les alkyl($C_8$-$C_{20}$)bétaïnes, les sulfobétaïnes, les alkyl($C_8$-$C_{20}$) amido alkyl($C_1$-$C_6$)bétaïnes et les alkyl-($C_8$-$C_{20}$)amido alkyl($C_1$-$C_6$) sulfobétaïnes.

Parmi ces composés, on peut citer les produits vendus sous la dénomination "MIRANOL", tels que décrits dans des brevets US-A-2.528.378 et 2.781.354 et classés dans le dictionnaire CTFA, 3ème édition, 1982, sous la dénomination d'Amphocarboxyglycinates et Amphocarboxypropionates.

Les alkylbétaïnes sont choisies de préférence parmi les alkyl ($C_{10}$-$C_{20}$)bétaïnes.

Par suite de la présence de l'agent de surface, les compositions sont utilisées, par exemple, comme démaquillant pour le visage, la présence de dialkylphtalate apportant alors une sensation de confort très nette, c'est-à-dire que l'on ne sent ni tiraillement, ni dessèchement, contrairement à ce qu'il se passe habituellement lorsque l'on utilise les solutions aqueuses contenant un tensio-actif.

Selon un mode de réalisation de l'invention, la composition biphasée renferme également des particules solides insolubles aussi bien dans la phase aqueuse que dans la phase huileuse contenant le dialkylphtalate, particules qui se rassemblent à l'interface des billes de la phase huileuse et de la phase aqueuse et contribuent à augmenter la stabilité de la dispersion des micro-billes de dialkylphtalate dans la phase aqueuse. Ces particules solides doivent être de petite dimension. Généralement, elles ont une dimension inférieure à 10 $\mu$m.

Les particules solides insolubles sont choisies de préférence dans le groupe formé par les matières minérales et organiques suivantes : oxyde de fer, dioxyde de titane, oxyde d'antimoine, oxyde de magnésium, alumine, oxyde de zinc, peroxyde de zinc, aluminate de calcium, acide silicique, silico-aluminate de magnésium, talc, mica, kaolin colloïdal, bentonite, laurate de zinc, chlorure de polyvinyle, nacre, noir de carbone, lanoline.

La composition selon l'invention peut également renfermer des adjuvants habituellement utilisés dans la cosmétique. Selon leur nature hydrophile ou lipophile, ces adjuvants seront dissous dans la phase aqueuse ou dans la phase huileuse.

Parmi ces adjuvants, on peut citer les parfums, les agents conservateurs, les colorants, les agents adoucissants tels que l'allantoïne; les extraits de plantes; les extraits de fruits; les agents hydratants tels que la glycérine; l'hydroxyproline; les agents tampons, les humectants tels que le butylèneglycol; les huiles, les vitamines, notamment la vitamine B5, la vitamine E, les séquestrants tels que l'acide éthylène diamine tétracétique (EDTA), les agents d'éclat du teint tels que la guanosine, les filtres UV, etc.

Pour préparer la composition à deux phases, on prépare d'abord la phase aqueuse en dissolvant dans l'eau les adjuvants solubles dans l'eau. S'il y a lieu, on disperse dans cette phase aqueuse, soit à froid, soit en chauffant légèrement, les particules solides insolubles dans l'eau et on y verse ensuite la phase huileuse contenant le dialkylphtalate et, s'il y a lieu, les produits miscibles avec le dialkylphtalate et les adjuvants liposolubles (huile, parfum, etc.). On agite environ une heure à une heure et demi et en laissant reposer on obtient une séparation en deux phases.

L'invention sera mieux comprise à l'aide des exemples non limitatifs ci-après.

## EXEMPLE 1

### DEMAQUILLANT A EFFET POUDREUX

| | | % en poids |
|---|---|---|
| - Dibutylphtalate | | 10 |
| - Parfum | | 0,05 |
| - Nacre | | 0,01 |
| - Hydroxyéthyl-1 lauryl-2 carboxyméthyl-3 imidazolinium | | 2 |
| - Acide 2-hydroxy 4-méthoxybenzophénone 5-sulfonique (Filtre UV), vendu sous la dénomination UVINUL MS 540 par BASF | | 0,05 |
| - Acide éthylènediamine tétracétique (EDTA) | | 0,05 |
| - Conservateur | | 0,1 |
| - Colorant | | 0,1 |
| - Eau | qsp | 100 |

## EXEMPLE 2

### DEMAQUILLANT A EFFET POUDREUX

| | | % en poids |
|---|---|---|
| - Diméthylphtalate | | 5 |
| - Parfum | | 0,5 |
| - Glycérine | | 3,0 |
| - Hydroxyproline | | 1 |
| - Vitamine B5 | | 1 |
| - Hydroxyéthyl-1 lauryl-2 carboxyméthyl-3 imidazolinium | | 4 |
| - Guanosine | | 0,01 |
| - Acide éthylène diamine tétracétique (EDTA) | | 0,05 |
| - Acide 2-hydroxy 4-méthoxybenzophénone 5-sulfonique (Filtre UV) | | 0,05 |
| - Conservateur | | 0,2 |
| - Eau | qsp | 100 |

## EXEMPLE 3

DEMAQUILLANT A EFFET POUDREUX

| | | % en poids |
|---|---|---|
| - Diéthylphtalate | | 4 |
| - Parfum | | 1 |
| - Butylèneglycol | | 2 |
| - Glycérine | | 3 |
| - Acide citrique (Tampon) | | 2 |
| - Extraits de fruits | | 5 |
| - Lauryl éther sulfate de sodium | | 3 |
| - Conservateur | | 0,3 |
| - Eau | qsp | 100 |

## EXEMPLE 4

### DEMAQUILLANT A EFFET POUDREUX

| | | % en poids |
|---|---|---|
| - Diéthylphtalate | | 15 |
| - Dioctyladipate | | 10 |
| - Nacre | | 0,01 |
| - Cocoamphocarboxyglycinate vendu sous la dénomination MIRANOL C2M par la Société MIRANOL | | 3,3 |
| - Alkyléthersulfate vendu sous la dénomination TEXAPON ASV par la Société HENKEL | | 2,7 |
| - Conservateurs | | 0,2 |
| - Benzoate de sodium | | 2 |
| - Acide 2-hydroxy 4-méthoxybenzophénone 5-sulfonique (Filtre UV), vendu sous la dénomination UVINUL MS 540 par la Société BASF | | 0,05 |
| - Sel disodique de l'acide éthylène diamine tétracétique (EDTA) | | 0,05 |
| - Eau | qsp | 100 |

**Revendications**

**1.** Composition cosmétique liquide nettoyante à deux phases, constituée d'une phase aqueuse et d'une phase huileuse, caractérisée en ce que :

(i) la phase aqueuse renferme un ou plusieurs agents de surface choisi dans le groupe formé par les agents de surface anioniques, amphotères, zwittérioniques et leurs mélanges;

(ii) la phase huileuse est constituée de 50% à 100% en poids d'un ou plusieurs dialkylphtalates, de formule générale :

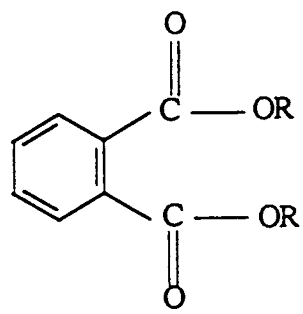

où R est un reste alkyle en $C_1$-$C_4$;

lorsque la teneur en dialkylphtalate est inférieure à 100% en poids, le complément à 100% est constitué d'un ou plusieurs produits miscibles au dialkylphtalate;

(iii) la phase huileuse se présente sous forme de micro-billes qui, par agitation, se mettent en suspension dans la phase aqueuse et au repos se déposent au fond de la phase aqueuse

la composition étant exempte d'alcool.

**2.** Composition selon la revendication 1, caractérisée en ce que la phase huileuse est constituée de 100% en poids d'un ou plusieurs dialkylphtalates.

**3.** Composition selon la revendication 1 ou 2, caractérisée en ce qu'elle contient de 0,5 à 20% en poids de dialkylphtalate du poids total de la composition.

**4.** Composition selon la revendication 3, caractérisée en ce qu'elle contient de 2 à 15% en poids de dialkylphtalate.

**5.** Composition selon la revendication 3, caractérisée en ce que l'agent de surface anionique est choisi dans le groupe formé par les acyliséthionates, les acyl méthyl taurates, les sels alcalins, les sels de magnésium, d'ammonium, d'amines ou d'amino alcools des composés suivants :

- les alkylsulfates, les alkyléthersulfates, les alkylamidoéthersulfates, les alkylarylpolyéthersulfates, les monoglycérides sulfates;
- les alkylsulfonates, les alkylamides sulfonates, les alkylaryl sulfonates, les oléfines sulfonates, les paraffines sulfonates;
- les alkylsulfosuccinates, les alkyléthersulfosuccinates, les alkylamide-sulfosuccinates;
- les alkylsulfosuccinamates, les alkylsulfoacétates;
- les alkylphosphates, les alkylétherphosphates;
- les acylsarcosinates;
- les N-acylpolypeptides,

dont le radical alkyle ou acyle est constitué par une chaîne carbonée comportant de 12 à 20 atomes de carbone;

les sels des acides oléique, ricinoléique, palmitique, stéarique; des acides d'huile de coprah, des acides d'huile de coprah hydrogénée; les acyl lactylates, dont le radical acyle comporte de 8 à 20 atomes de carbone;

les acides éthers carboxyliques polyoxyalkylénés répondant à la formule :

$$R\text{-}(OC_3H_6)_p\text{-}(OC_2H_4)_n\text{-}OCH_2COOA$$

dans laquelle R désigne un radical alkyle ou un mélange de radicaux alkyle ou alcényle en $C_8$-$C_{22}$, linéaire ou ramifié, un radical alkyl ($C_8$-$C_9$)phényle, R'CONH-$CH_2$-$CH_2$- avec R' désignant un radical alkyle ou alcényle, linéaire ou ramifié, en $C_{11}$-$C_{21}$; n est un nombre entier ou décimal compris entre 2 et 24; p est un nombre entier ou décimal compris entre 0 et 6; A désigne un atome d'hydrogène ou bien Na, K, Li, 1/2 Mg ou un reste monoéthanolamine, ammonium ou triéthanolamine.

**6.** Composition selon la revendication 3, caractérisée en ce que l'agent de surface amphotère ou zwittérionique est choisi dans le groupe formé par :

- les dérivés d'amines secondaires ou tertiaires aliphatiques, dans lesquels le radical aliphatique est une chaîne linéaire ou ramifiée comportant de 8 à 18 atomes de carbone et qui contient au moins un groupe anionique hydrosolubilisant carboxylique, sulfonate, sulfate, phosphate ou phosphonate;
- les alkyl($C_8$-$C_{20}$)imidazolinium carboxyméthyle;
- les N-alkyl($C_8$-$C_{20}$)-$\beta$-iminodipropionates;
- les alkyl($C_8$-$C_{20}$)bétaïnes, les sulfobétaïnes, les alkyl($C_8$-$C_{20}$) amido alkyl($C_1$-$C_6$)bétaïnes et les alkyl($C_8$-$C_{20}$)amido alkyl($C_1$-$C_6$) sulfobétaïnes.

**7.** Composition selon l'une quelconque des revendications 1 à 6, caractérisée en ce que l'agent de surface est présent dans la phase aqueuse en une quantité de 0,1 à 30% en poids du poids total de la composition.

**8.** Composition selon la revendication 7, caractérisée en ce que l'agent de surface est présent en une quantité de 2 à 6% en poids du poids total de la composition.

**9.** Composition selon l'une quelconque des revendications 1 à 8, caractérisée en ce que la phase aqueuse représente de 74 à 99,5% en poids du poids total de la composition.

**10.** Composition selon la revendication 9, caractérisée en ce que la phase aqueuse représente de 90 à 95% en poids du poids total de la composition.

**11.** Composition selon l'une quelconque des revendications 1 à 10, caractérisée en ce qu'elle renferme au moins un adjuvant choisi dans le groupe formé par les parfums, les agents conservateurs, les colorants, les agents adoucissants, les extraits de plantes, les extraits de fruits, les agents hydratants, les agents tampon, les agents humectants, les huiles, les vitamines, les agents d'éclat du teint, les filtres UV, les séquestrants.

**12.** Composition selon l'une quelconque des revendications 1 à 11, caractérisée en ce qu'elle renferme également des particules solides insolubles dans la phase aqueuse et dans la phase huileuse, lesdites particules étant rassemblées à l'interface huile/eau.

**Claims**

**1.** Two-phase liquid cleansing cosmetic composition consisting of an aqueous phase and an oily phase characterised in that :

(ii) the aqueous phase contains one or more surface-active agents chosen from the goup consisting of anionic amphoteric or zwitterionic surface-active agents and their mixtures;

(ii) the oily phase consists of 50% to 100% by weight of one or more dialkylphtalates of generale formula :

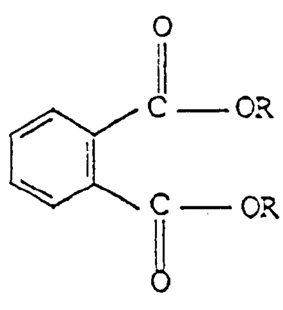

in which R is a $C_1$-$C_4$ alkyl residue;

(iii) the oily phase is in the form of microbeads which, on stirring , become suspended in the aqueous phase and, on standing, settle at the bottom of the aqueous phase, the composition being free of alcohol.

**2.** Composition according to Claim 1, characterised in that the oily phase consists of 100% by weight of one or more dialkylphtalates.

**3.** Composition according to Claim 1 or 2 , characterised in that it contains 0.5 to 20% by weight of dialkylphtalate of the total weight of the composition.

**4.** Composition according to Claim 3, characterised in that it contains 2 to 10% by weight of dialkylphtalate.

**5.** Composition according to any one of Claims 1 to 4, characterised in that the anionic surface-active agent is chosen from the group consisting of acylisethionates,acylmethyltaurates , alkali metal salts, magnesium, ammonium, amine or aminoalcohol salts of the following compounds :

- alkylsulphates, alkylethersulphates, alkylamidoethersulphates, alkylarylpolyethersulphates, monoglyceridesulphates;
- alkylsulphonates, alkylamidesulphonates, alkylarylsulphonates, olefinsulphonates, paraffinsulphonates;
- alkylsulphosuccinates, alkylethersulphosuccinates, alkylamidesulphosuccinates;
- alkylsulphosuccinamates, alkylsulphoacetates;
- alkylphosphates, alkyletherphosphates;
- acylsarcosinates;
- N-acylpolypeptides.

whose alkyl or acyl radical consists of a carbon chain comprising 12 to 20 carbon atoms;

the salts of oleic, ricinoleic, palmitic or stearic acids, of coprah oil acids, of hydrogenated coprah oil acids; and acyllactylates whose acyl radical comprises 8 to 20 carbon atoms.

the polyoxyalkylene-containing carboxylic ether acids of the formula:

$$R-(OC_3H_6)_p-(OC_2H_4)_n-OCH_2COOA$$

in which R denotes an alkyl radical or a mixture of $C_8$-$C_{22}$, linear or branched, alkyl or alkenyl radicals, a ($C_8$-$C_9$ alkyl)phenyl or $R'CONH-CH_2-CH_2-$ radical, with R' denoting a $C_{11}$-$C_{21}$, linear or branched alkyl or alkenyl radical; n is an integer or a decimal fraction between 2 and 24, p is an integer or a decimal fraction between 0 and 6, A denotes a hydrogen atom or, alternatively, Na, K, Li, 1/2 Mg or a monoethanolamine, ammonium or triethanolamine residue.

**6.** Composition according to Claim 3, characterised in that the amphoteric or zwitterionic surface-active agents is chosen from the group consisting of:

- aliphatic secondary or tertiary amine derivatives in which the aliphatic radical is a linear or branched chain comprising 8 to 18 carbon atoms and which contains at least one water-solubilising carboxylic, sulphonate, sulphate, phosphate or phosphonate anionic group;
- ($C_8$-$C_{20}$ alkyl)imidazoliniumcarboxymethyls;
- ($C_8$-$C_{20}$ N-alkyl)-$\beta$-iminodipropionates;
- ($C_8$-$C_{20}$ alkyl)betaines, sulphobetaines, ($C_8$-$C_{20}$ alkyl)amido($C_1$-$C_6$ alkyl)betaines and ($C_8$-$C_{20}$ alkyl)amido($C_1$-$C_6$ alkyl)sulphobetaines.

**7.** Composition according to any one of Claims 1 to 6, characterised in that the surface-active agent is present in the aqueous phase in an amount of 0.1 to 30% by weight of the total weight of the composition.

**8.** Composition according to Claim 7, characterized in that the surface-active agent is present in an amount of 2 to 6% by weight of the total weight of the composition.

**9.** Composition according to any one of Claims 1 to 8, characterized in that the aqueous phase represents 74 to 99.5% by weight of the total weight of the composition.

**10.** Composition according to Claim 9, characterized in that the aqueous phase represents 90 to 95% by weight of the total weight of the composition.

**11.** Composition according to any one of Claims 1 to 10, characterized in that it contains at least one adjuvant chosen from the group consisting of perfumes, preserving agents, colorants, softening agents, plant extracts, fruit extracts, moisturizing agents, buffers, humectants, oils, vitamins, complexion-enhancing agents, UV screens and sequestrants.

**12.** Composition according to any one of Claims 1 to 11, characterized in that it also contains solid particles which are insoluble in the aqueous phase and in the oily phase, the said particles being collected at the oil/water interface.

## Patentansprüche

**1.** Zweiphasige flüssige kosmetische Reinigungsmittel-Zusammensetzung aus einer Wasser-Phase und einer Ölphase,
dadurch **gekennzeichnet**, daß
(i) die wässrige Phase eines oder mehrere Oberflächenmittel aus der Gruppe aus anionischen, amphoteren, zwitterionischen Oberflächenmitteln und aus deren Mischungen enthält;
(ii) die ölige Phase aus 50 bis 100 Gew.% eines oder mehrerer Dialkylphthalate der allgemeinen Formel zusammengesetzt ist:

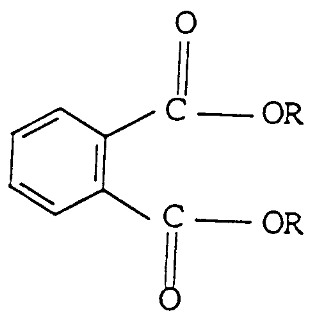

worin R ein $C_{1-4}$-Alkylrest ist;
und wenn der Gehalt an Dialkylphthalat unterhalb 100 Gew.% liegt, der Rest auf 100% aus einem oder mehreren mit Dialkylphthalat mischbaren Produkten zusammengesetzt ist;
(iii) die ölige Phase in Form von Mikrokugeln vorliegt, die sich, durch einen Rührvorgang, in der wässrigen Phase suspendieren lassen und bei Beruhigung am Boden der wässrigen Phase absetzen,
wobei die Zusammensetzung unter Ausschluß von Alkohol gebildet ist.

**2.** Zusammensetzung gemäß Anspruch 1,
dadurch **gekennzeichnet**, daß
die ölige Phase aus 100 Gew.% eines oder mehrerer Dialkylphthalate zusammengesetzt ist.

**3.** Zusammensetzung gemäß Anspruch 1 oder 2,
dadurch **gekennzeichnet**, daß
sie 0,5 bis 20 Gew.% Dialkylphthalat, bezogen auf Gesamtgewicht der Zusammensetzung, enthält.

**4.** Zusammensetzung gemäß Anspruch 3,
dadurch **gekennzeichnet**, daß
sie 2 bis 15 Gew.% Dialkylphthalat enthält.

**5.** Zusammensetzung gemäß Anspruch 3,
dadurch **gekennzeichnet**, daß
das anionische Oberflächenmittel aus der Gruppe aus Acylisethionaten, Acylmethyltauraten, Alkalisalzen, Magnesium-, Ammonium-, Amin- oder Aminoalkoholsalzen der folgenden Verbindungen:
- von Alkylsulfaten, Alkylethersulfaten, Alkylamidoethersulfaten, Alkylarylpolyethersulfaten, Monoglyceridsulfaten;
- von Alkylsulfonaten, Alkylamidsulfonaten, Alkylarylsulfonaten, Olefinsulfonaten, Paraffinsulfonaten;
- von Alkylsulfosuccinaten, Alkylethersulfosuccinaten, Alkylamidsulfosuccinaten;
- von Alkylsulfosuccinamaten, Alkylsulfoacetaten;
- von Alkylphosphaten, Alkyletherphosphaten;
- von Acylsarcosinaten;

- von N-Acylpolypeptiden, deren Alkyl- oder Acylrest aus einer Kohlenstoffkette mit 12 bis 20 Kohlenstoffatomen zusammengesetzt sind;

aus Salzen der Öl-, Rizinolein-, Palmitin-, Stearinsäure; aus Säuren vom Öl von Koprah, Säuren vom Öl von hydriertem Koprah; aus Acyllactylaten, deren Acylrest 8 bis 20 Kohlenstoffatome enthält;
aus polyoxalkylierten Carboxylsäureethern der Formel:

$R\text{-}(OC_3H_6)_p\text{-}(OC_2H_4)_n\text{-}OCH_2\text{-}COOA$

worin R einen linearen oder verzweigten $C_{8-22}$-Alkylrest oder eine Mischung der Alkylreste oder einen entsprechenden Alkenylrest, einen $C_{8-9}$-Alkylphenylrest, einen $R'CONH\text{-}CH_2\text{-}CH_2$-Rest darstellt, worin R' einen linearen oder verzweigten $C_{11-21}$-Alkyl- oder -Alkenylrest darstellt,
n eine ganze Zahl oder Dezimalzahl von 2 bis 24 ist,
p eine ganze Zahl oder Dezimalzahl von 0 bis 6 ist,
A ein Wasserstoffatom oder auch Na, K, Li, 1/2 Mg oder einen Monoethanolamin-, Ammonium- oder Triethanolaminrest darstellt,
ausgewählt ist.

**6.** Zusammensetzung gemäß Anspruch 3,
dadurch **gekennzeichnet**, daß
das amphotere oder zwitterionische Oberflächenmittel ausgewählt ist aus der Gruppe aus:
- sekundären oder tertiären aliphatischen Aminderivaten, in denen der aliphatische Rest eine lineare oder verzweigte Kette mit 8 bis 18 Kohlenstoffatomen ist und mindestens eine anionische wasserlösliche Carboxyl-, Sulfonat-, Sulfat-, Phosphat- oder Phosphonatgruppe enthält;
- $C_{8-20}$-Alkylimidazoliniumcarboxymethyl;
- N-$C_{8-20}$-Alkyliminodipropionaten;
- $C_{8-20}$-Alkylbetainen, Sulfobetainen, $C_{8-20}$-Alkylamido-$c_{1-6}$-alkylbetainen und $C_{8-20}$-Alkylamido-$C_{1-6}$-alkylzulfobetainen.

**7.** Zusammensetzung gemäß einem jeden der Ansprüche 1 bis 6,
dadurch **gekennzeichnet**, daß
das Oberflächenmittel in der wässrigen Phase in einer Menge von 0,1 bis 30 Gew.% des Gesamtgewichts der Zusammensetzung vorliegt.

**8.** Zusammensetzung gemäß Anspruch 7,
dadurch **gekennzeichnet**, daß
das Oberflächenmittel in einer Menge von 2 bis 6 Gew.% des Gesamtgewichts der Zusammensetzung vorhanden ist.

**9.** Zusammensetzung gemäß einem jeden der Ansprüche 1 bis 8,
dadurch **gekennzeichnet**, daß
die wässrige Phase 74 bis 99,5 Gew.% des Gesamtgewichts der Zusammensetzung darstellt.

**10.** Zusammensetzung gemäß Anspruch 9,
dadurch **gekennzeichnet**, daß
die wässrige Phase 90 bis 95 Gew.% des Gesamtgewichts der Zusammensetzung darstellt.

**11.** Zusammensetzung gemäß einem jeden der Ansprüche 1 bis 10,
dadurch **gekennzeichnet**, daß
sie mindestens einen Hilfstoff enthält, ausgewählt aus der Gruppe, gebildet aus Parfüm-Produkten, Konservierungsmitteln, Färbemitteln, weichmachenden Mitteln, Pflanzenextrakten, Fruchtextrakten, hydratisierenden Mitteln, Puffermitteln, Feuchtigkeitsmitteln, Ölen, Vitaminen, Glanzmitteln für den Teint, UV-Filterstoffen, Sequestriermitteln.

**12.** Zusammensetzung gemäß einem jeden der Ansprüche 1 bis 11,
dadurch **gekennzeichnet**, daß
sie auch Feststoffpartikel enthält, die in der wässrigen und öligen Phase unlöslich sind, wobei die genannten Partikel in der Zwischenphase Öl/Wasser angeordnet sind.